# FASCICULE DE BREVET EUROPEEN

(11) **EP 2 151 428 B1**
(45) Date de publication et mention de la délivrance du brevet: **07.03.2012**
(21) Numéro de dépôt: 09290606.4
(22) Date de dépôt: 04.08.2009
(51) Int. Cl.: C07C 231/02, C07C 231/12, C07C 233/11, C07C 233/18, C07C 233/91, C07D 209/48

(54) **Nouveau procédé de synthèse de l'agomélatine**
Verfahren zur Herstellung von Agomelatin
Process for the preparation of agomelatin

(30) Priorité: 05.08.2008 FR 0804465
(43) Date de publication de la demande: 10.02.2010
(73) Titulaire: Les Laboratoires Servier, 92284 Suresnes Cedex (FR)
(72) Inventeur: Hardouin, Christophe, 76600 Le Havre (FR); Lecouve, Jean-Pierre, 76600 Le Havre (FR); Bragnier, Nicolas, 76000 Rouen (FR)

(56) Documents cités:
- EP-A- 0 447 285
- EP-A- 0 962 434
- EP-A- 0 994 102
- EP-A- 1 564 202
- WO-A1-2010/012208

## Description

La présente invention concerne un nouveau procédé de synthèse industriel de l'agomélatine ou *N*-[2-(7-méthoxy-1-naphtyl)éthyl]acétamide de formule (I) :

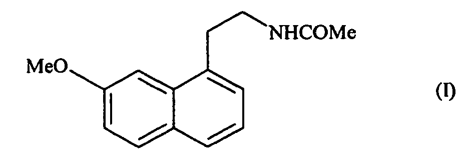

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202. Par ailleurs, la demande WO 2010/012208 décrit une voie d'accès de l'agomélatine impliquant le 2-[2-(7-méthoxy-1-naphtyl)éthyl]-1*H*-isoindole-1,3(2*H*)-dione, obtenu à partir d'un dérivé de 2-(7-méthoxy-1-naphtyl)éthylsulfonate. Quant au document EP 0 962 434, il décrit la formation d'oléfines aromatiques, présentant notamment un noyau naphtalénique, à l'aide de catalyseurs au palladium contenant des ligands de type phosphite.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone avec un rendement moyen inférieur à 30%.

Dans le brevet EP 1 564 202, la demanderesse a mis au point une nouvelle voie de synthèse beaucoup plus performante et industrialisable, en seulement quatre étapes à partir de la 7-méthoxy-1-tétralone, et permettant d'obtenir l'agomélatine de façon très reproductible sous une forme cristalline bien définie.

Toutefois, la recherche de nouvelles voies de synthèse, en particulier à partir de matières premières moins onéreuses que la 7-méthoxy-1-tétralone, est toujours d'actualité.

La demanderesse a poursuivi ses investigations et mis au point un nouveau procédé de synthèse de l'agomélatine à partir du 7-méthoxy-1-naphtol : cette nouvelle matière première présente l'avantage d'être simple, aisément accessible en grandes quantités avec des coûts moindres. Le 7-méthoxy-1-naphtol présente également l'avantage de posséder dans sa structure un noyau naphtalène, ce qui évite d'intégrer dans la synthèse une étape d'aromatisation, étape toujours délicate d'un point de vue industriel.

Ce nouveau procédé permet par ailleurs d'obtenir l'agomélatine de façon reproductible et sans nécessiter de purification laborieuse, avec une pureté qui est compatible avec son utilisation comme principe actif pharmaceutique.

Plus spécifiquement, la présente invention concerne un procédé de synthèse industrielle du composé de formule (I) : 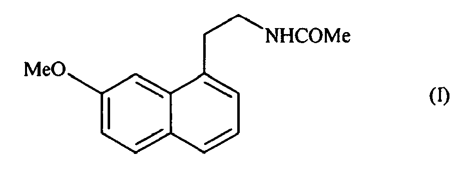 caractérisé en ce que l'on met en réaction le 7-méthoxy-1-naphtol de formule (II) : 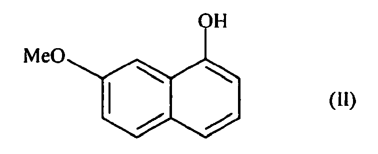 sur lequel on condense en présence de palladium, après transformation de la fonction hydroxy en groupe partant comme le groupement halogène, tosylate ou trifluorométhanesulfonate, le composé de formule (III) : CH₂=CH-R (III)
dans laquelle R représente le groupement 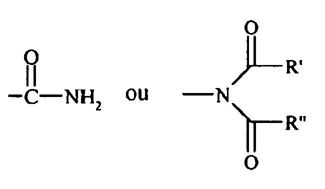 où R' et R", identiques ou différents, représentent chacun un groupement alkyle (C₁-C₆) linéaire ou ramifié, ou R' et R" forment ensemble une chaîne alkylène (C₂-C₃), le cycle ainsi formé pouvant être fusionné à un phényle,
pour conduire au composé de formule (IV) : 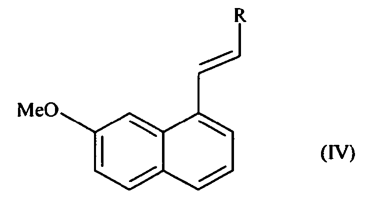 dans laquelle R est tel que défini précédemment,
que l'on soumet à une hydrogénation catalytique pour conduire au composé de formule (V) : 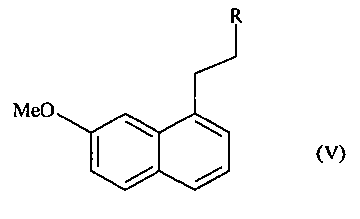 dans laquelle R est tel que défini précédemment,
que l'on soumet à une hydrolyse basique ou acide, ou à un système binaire réducteur/acide pour conduire au composé de formule (VI) ou à son chlorhydrate: 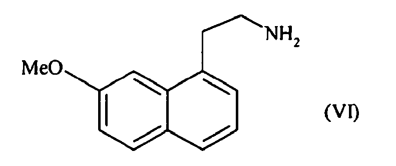 composé de formule (VI) qui est successivement soumis à l'action d'acétate de sodium puis d'anhydride acétique pour conduire au composé de formule (I) que l'on isole sous la forme d'un solide.

De façon préférentielle, le composé de formule (III) selon le procédé de l'invention est un dérivé du phtalimide, et plus préférentiellement le *N*-vinylphtalimide. Avantageusement, le composé de formule (III) représente également l'acrylamide.

Avantageusement la condensation selon l'invention du composé de formule (III) pour obtenir le composé de formule (IV) est réalisée avec du palladium tetrakis triphénylphosphine ; la réaction est réalisée préférentiellement à reflux de toluène.

L'hydrogénation du composé de formule (IV) en composé de formule (V) est réalisée de préférence avec du palladium sur charbon, et plus particulièrement avec du palladium sur charbon contenant au minimum 5% de palladium.

De façon avantageuse, l'hydrolyse du composé de formule (V) est réalisée préférentiellement avec un système binaire réducteur/acide comme NaBH₄ puis acide acétique par exemple, ou, lorsque R représente un groupement C(O)NH₂ la transformation du composé de formule (V) en composé de formule (VI) est préférentiellement réalisé avec une base comme NaOBr ou NaOCl par exemple.

Ce procédé est particulièrement intéressant pour les raisons suivantes :
- il permet d'obtenir à l'échelle industrielle le composé de formule (I) avec d'excellents rendements à partir d'une matière première simple et peu onéreuse ;
- les conditions opératoires mises au point selon l'invention permettent de totalement contrôler la régiosélectivité lors du couplage avec le composé de formule (III);
- il permet d'éviter une réaction d'aromatisation puisque le noyau naphtalénique est présent dans le substrat de départ ;
- enfin, le composé de formule (I) obtenu présente de façon reproductible les caractéristiques de la forme cristalline décrite dans le brevet EP1564202.

Les composés de formules (IV) obtenus selon le procédé de l'invention sont nouveaux et utiles en tant qu'intermédiaires de synthèse de l'agomélatine dans laquelle ils sont soumis à une réaction de réduction puis d'hydrolyse puis de couplage avec l'anhydride acétique.

Les exemples ci-dessous illustrent l'invention, mais ne la limitent en aucune façon.

### Exemple 1 : N-[2-(7-Méthoxy-1-naphtyl)éthyl]acétamide

### Stade A : 7-Méthoxy-1-naphtyl trifluorométhanesulfonate

Dans un réacteur sont introduits 2,7 g de 7-méthoxy-1-naphtol, 1,1 éq d'anhydride triflique et 1,1 éq de 2,6-di-tertbutyl-4-méthyl-pyridine dans le dichlorométhane (45 ml). Le mélange est porté au reflux pendant 12 heures, puis filtré, et les jus sont lavés avec une solution de HCl 1N puis NaCl saturée. La phase organique est évaporée et le résidu obtenu est purifié par chromatographie sur gel de silice (éluant CH₂Cl₂/méthyl cyclohexane 1/9) pour conduire au produit du titre sous la forme d'une huile avec un rendement de 91% et une pureté chimique supérieure à 99%.

### Stade B : 2-[2-(7-Méthoxy-1-naphtyl)éthènyl]-1H-isoindole-1,3(2H)-dione

Dans un réacteur sont introduits 2 g du composé obtenu dans le Stade A, 2 éq de N-vinylphtalimide, 1,25 éq de diisopropyléthylamine et 0,05 éq de palladium tétrakis triphénylphosphine dans du toluène et portés à reflux. La réaction se poursuit à reflux pendant 12 heures puis le milieu réactionnel est refroidi à l'ambiante. De l'acétate d'éthyle est ajouté, puis des lavages avec de l'eau et une solution de HCl 1N sont effectués. Après évaporation des solvants, le résidu obtenu est purifié par chromatographie sur gel de silice (éluant dichlorométhane/heptane 1/1, puis dichlorométhane) pour conduire au produit du titre avec un rendement de 80% et une pureté chimique supérieure à 95%.
*Point de fusion : 146°C*

### Stade C : 2-[2-(7-Méthoxy-1-naphtyl)éthyl]-1H-isoindole-1,3(2H)-dione

Dans un réacteur sont introduits dans un mélange méthanol/THF 1/2, 2 g du composé obtenu dans le Stade B et 1 g de palladium sur charbon à 5%, sous hydrogène à pression et température ambiantes. Après 8 heures de réaction, le milieu réactionnel est filtré. Après évaporation des solvants, le produit du titre est obtenu quantitativement avec une pureté chimique de 95%.
*Point de fusion : 154°C*

### Stade D : 2-(7-Méthoxy-1-naphtyl)éthanamine

Dans un réacteur sont introduits dans un mélange 2-propanol/eau 6/1, 1 g du composé obtenu dans le Stade C, 5 éq de NaBH₄, et le mélange est agité à l'ambiante. Puis de l'acide acétique (0,2 éq) est ajouté et le milieu réactionnel est porté à 80°C pendant 8 heures. Après évaporation des solvants et co-évaporation de l'eau avec du toluène, le résidu brut obtenu est directement engagé dans la réaction d'acétylation sans plus de purification.

### Stade E : N-[2-(7-Méthoxy-1-naphtyl)éthyl]acétamide

Dans un réacteur sont introduits 5 g du composé obtenu au stade D et 2 g d'acétate de sodium dans de l'éthanol. Le milieu est agité puis 2,3 g d'anhydride acétique sont additionnés, le milieu réactionnel est porté à reflux et 20 ml d'eau sont ajoutés. La réaction est laissée revenir à l'ambiante et le précipité obtenu est filtré, lavé par un mélange éthanol/eau 35/65 pour conduire au produit du titre avec un rendement de 80% pour les deux étapes D et E et une pureté chimique supérieure à 99%.
*Point de fusion : 108°C*

### Exemple 2 : N-[2-(7-Méthoxy-1-naphtyl)éthyl]acétamide

### Stade A : 3-(7-Méthoxy-1-naphtyl)-2-propènamide

Une solution du composé obtenu dans le Stade A de l'Exemple 1 (12,1 g) dans 80 mL de DMF est dégazée par bullage d'azote pendant 10 minutes à 20°C. A cette solution sont additionnés successivement de la triéthylamine (6,6 ml), de l'acrylamide (5,6 g), de la neocuproine hydratée (454 mg) et Pd(OAc)₂ (445 mg).

Le milieu est chauffé pendant 1 h à 100°C puis laissé refroidir à 20°C. Après dilution dans AcOEt (100 ml) puis addition d'une solution saturée de NH₄Cl, les phases sont séparées. La phase organique est concentrée sous pression réduite puis le résidu est repris avec 50 ml d'AcOEt. Le précipité est filtré pour conduire au produit du titre sous la forme d'une poudre.

### Stade B : 3-(7-Méthoxy-1-naphtyl)propanamide

A une solution du composé obtenu dans le Stade A (0,5 g) dans un mélange MeOH (6,5 mL) / THF (6,5 mL) est additionné 0,12 g de Pd/C 5% (50% humide). Le milieu est purgé à l'azote puis à l'hydrogène avant d'être chauffé à 50°C sous pression atmosphérique pendant 1 heure. La suspension est alors filtrée sur célite et le filtre est lavé par un mélange MeOH (5 mL) / THF (5 mL). Les jus sont concentrés sous pression réduite pour conduire au produit du titre sous la forme d'un solide qui est engagé dans l'étape suivante sans purification supplémentaire.

### Stade C : 2-(7-Méthoxy-1-naphtyl)éthanamine, chlorhydrate

A une solution eau (3 mL) / acétonitrile (3 mL) est additionné l'iodosobenzène diacétate (0,88 g). Après 10 minutes d'agitation à 20°C, le composé obtenu dans le Stade B (500 mg) est additionné par portions puis le mélange est laissé à 20°C pendant 2 h. Après consommation de la matière première, l'acétonitrile est distillé sous pression réduite. Le résidu est repris dans l'eau (10 mL) puis traité par une solution concentrée de HCl (0,4 mL). Après filtration, le précipité obtenu est lavé par de l'acétate d'éthyle puis séché en étuve pour conduire au produit du titre.
*Point de fusion : 243°C*

### Stade D : N-[2-(7-Méthoxy-1-naphtyl)éthyl]acétamide

Dans un réacteur sont introduits 5 g du composé obtenu au stade C et 2 g d'acétate de sodium dans de l'éthanol. Le milieu est agité puis 2,3 g d'anhydride acétique sont additionnés, le milieu réactionnel est porté à reflux et 20 ml d'eau sont ajoutés. La réaction est laissée revenir à l'ambiante et le précipité obtenu est filtré, lavé par un mélange éthanol/eau 35/65 pour conduire au produit du titre.
*Point de fusion: 108°C*

### Exemple 3 : Détermination de la forme cristalline du composé N-[2-(7-méthoxy-1-naphtyl)éthyl]acétamide obtenu dans les Exemples 1 et 2

L'enregistrement des données a été effectué sur le diffractomètre haute résolution D8 de Bruker AXS avec les paramètres suivants : un domaine angulaire 3°-90° en 2θ, un pas de 0,01° et 30 s par pas. La poudre de *N*-[2-(7-méthoxy-1-naphtyl)éthyl]acétamide obtenue dans les Exemples 1 ou 2 a été déposée sur un support pour un montage en transmission. La source de rayons X est un tube au cuivre (λCuK_{α1} = 1,54056 Å). Le montage comporte un monochromateur avant (cristal de Ge(111)) et un détecteur solide résolu en énergie (MXP-D1, Moxtec-SEPH).

Le composé est bien cristallisé : la largeur des raies à mi-hauteur est de l'ordre de 0,07° en 2θ. Les paramètres suivants ont ainsi été déterminés :
- maille cristalline monoclinique,
- paramètres de maille : a = 20,0903 Å, b = 9,3194 Å, c = 15,4796 Å, β = 108,667°
- groupe d'espace : P2₁/n
- nombre de molécules dans la maille : 8
- volume de la maille : Vₘₐᵢₗₗₑ = 2746,742 Å³
- densité : d = 1,13 g/cm³.

### Exemple 4 : Détermination de la forme cristalline du composé N-[2-(7-méthoxy-1-naphtyl)éthyl]acétamide obtenu dans les Exemples 1 et 2 par diagramme de diffraction X sur poudre

La forme cristalline du composé obtenu dans les Exemples 1 et 2 est caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| **Angle 2 thêta (°)** | **Distance d inter-réticulaire** **(Å)** | **Intensité (%)** |
|---|---|---|
| 9,26 | 9,544 | 23 |
| 10,50 | 8,419 | 13 |
| 15,34 | 5,771 | 24 |
| 17,15 | 5,165 | 100 |

## Revendications

1. Procédé de synthèse industrielle du composé de formule (I) 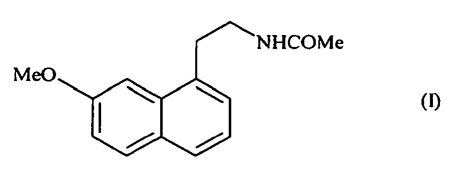 **caractérisé en ce que** l'on met en réaction le 7-méthoxy-1-naphtol de formule (II) : 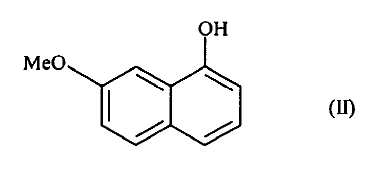 sur lequel on condense en présence de palladium, après transformation de la fonction hydroxy en groupe partant comme le groupement halogène, tosylate ou trifluorométhanesulfonate, le composé de formule (III) : CH₂=CH-R (III)
dans laquelle R représente le groupement 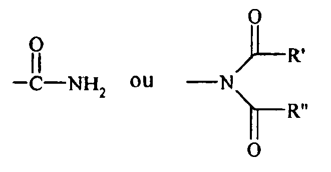 où R' et R", identiques ou différents, représentent chacun un groupement alkyle (C₁-C₆) linéaire ou ramifié, ou R' et R" forment ensemble une chaîne alkylène (C₂-C₃), le cycle ainsi formé pouvant être fusionné à un phényle,
pour conduire au composé de formule (IV) : 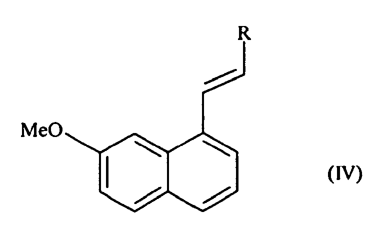 dans laquelle R est tel que défini précédemment,
que l'on soumet à une hydrogénation catalytique pour conduire au composé de formule (V) : 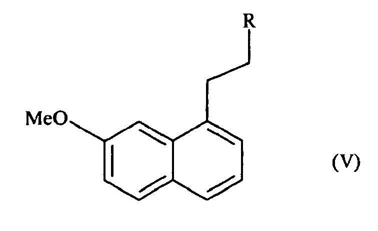 dans laquelle R est tel que défini précédemment,
que l'on soumet à une hydrolyse basique ou acide, ou à un système binaire réducteur/acide pour conduire au composé de formule (VI) ou à son chlorhydrate: 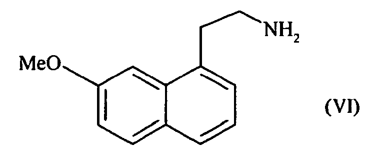 qui est successivement soumis à l'action d'acétate de sodium puis d'anhydride acétique pour conduire au composé de formule (I) que l'on isole sous la forme d'un solide.

2. Procédé de synthèse du composé de formule (I) selon la revendication 1 **caractérisé en ce que** le composé de formule (III) est le *N*-vinylphtalimide.

3. Procédé de synthèse du composé de formule (I) selon la revendication 1 **caractérisé en ce que** le composé de formule (III) est l'acrylamide.

4. Procédé de synthèse du composé de formule (I) selon la revendication 1, **caractérisé en ce que** la condensation du composé de formule (III) pour obtenir le composé de formule (IV) est réalisée avec du palladium tetrakis triphénylphosphine.

5. Composé de formule (IV) selon la revendication 1 utile en tant qu'intermédiaire de synthèse de l'agomélatine.

6. Utilisation du composé de formule (IV) selon la revendication 5 dans la synthèse de l'agomélatine.

7. Utilisation du composé de formule (II) selon la revendication 1 dans la synthèse de l'agomélatine.

8. Utilisation du composé de formule (V) selon la revendication 1 dans la synthèse de l'agomélatine, étant entendu que cette utilisation ne concerne pas le 2-[2-(7-méthoxy-1-naphtyl)éthyl]-1*H*-isoindole-1,3(2*H*)-dione.

9. Procédé de synthèse de l'agomélatine selon la revendication 1 à partir du composé de formule (IV) **caractérisé en ce que** le composé de formule (IV) est obtenu selon le procédé de synthèse selon l'une des revendications 1 à 4.

10. Procédé de synthèse de l'agomélatine selon la revendication 1 à partir du composé de formule (V) **caractérisé en ce que** le composé de formule (V) est obtenu selon le procédé de synthèse selon l'une des revendications 1 à 4.

11. Procédé de synthèse de l'agomélatine selon la revendication 1 à partir du composé de formule (VI) **caractérisé en ce que** le composé de formule (VI) est obtenu selon le procédé de synthèse selon l'une des revendications 1 à 4.

## Claims

1. Process for the industrial synthesis of the compound of formula (I) 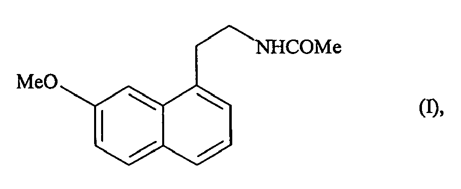 **characterised in that** there is reacted 7-methoxy-1-naphthol of formula (II): 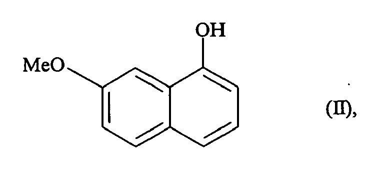 with which there is condensed, in the presence of palladium, after conversion of the hydroxy function into a leaving group such as a halogen, tosylate or trifluoromethanesulphonate group, the compound of formula (III): CH₂=CH-R (III),
wherein R represents the group 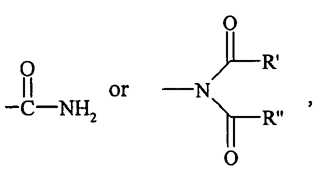 wherein R' and R", which may be the same or different, each represent a linear or branched (C₁-C₆)alkyl group or R' and R" together form a (C₂-C₃)alkylene chain, it being possible for the ring thereby formed to be fused with a phenyl group,
to yield the compound of formula (IV): 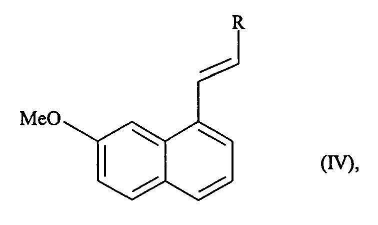 wherein R is as defmed hereinbefore,
which is subjected to catalytic hydrogenation to yield the compound of formula (V): 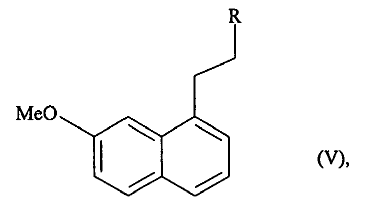 wherein R is as defined hereinbefore,
which is subjected to base or acid hydrolysis or to a binary reducing agent/acid system to yield the compound of formula (VI) or its hydrochloride: 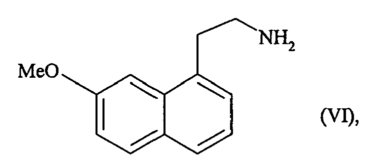 which is successively subjected to the action of sodium acetate and then acetic anhydride to yield the compound of formula (I), which is isolated in the form of a solid.

2. Process for the synthesis of the compound of formula (I) according to claim 1, **characterised in that** the compound of formula (III) is N-vinylphthalimide.

3. Process for the synthesis of the compound of formula (I) according to claim 1, **characterised in that** the compound of formula (III) is acrylamide.

4. Process for the synthesis of the compound of formula (I) according to claim 1, **characterised in that** the condensation of the compound of formula (III) to obtain the compound of formula (IV) is carried out using palladium tetrakis(triphenylphosphine).

5. Compound of formula (IV) according to claim 1 for use as an intermediate in the synthesis of agomelatine.

6. Use of the compound of formula (IV) according to claim 5 in the synthesis of agomelatine.

7. Use of the compound of formula (II) according to claim 1 in the synthesis of agomelatine.

8. Use of the compound of formula (V) according to claim 1 in the synthesis of agomelatine, with the proviso that this use does not relate to 2-[2-(7-methoxy-1-naphthyl)ethyl]-1*H*-isoindole-1,3(2*H*)-dione.

9. Process for the synthesis of agomelatine, according to claim 1, starting from the compound of formula (IV), **characterised in that** the compound of formula (IV) is obtained according to the synthesis process according to one of claims 1 to 4.

10. Process for the synthesis of agomelatine, according to claim 1, starting from the compound of formula (V), **characterised in that** the compound of formula (V) is obtained according to the synthesis process according to one of claims 1 to 4.

11. Process for the synthesis of agomelatine, according to claim 1, starting from the compound of formula (VI), **characterised in that** the compound of formula (VI) is obtained according to the synthesis process according to one of claims 1 to 4.

## Patentansprüche

1. Verfahren zur industriellen Synthese der Verbindung der Formel (I) 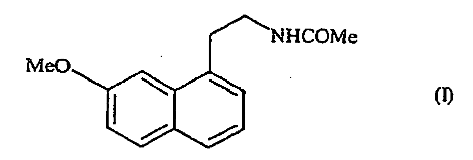 **dadurch gekennzeichnet, dass** man 7-Methoxy-1-naphthol der Formel (II): 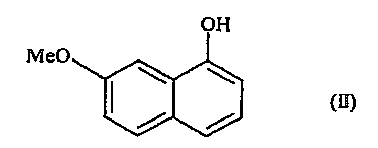 nach der Umwandlung der Hydroxygruppe in eine austretende Gruppe, wie ein Halogenatom, eine Tosylat- oder Trifluormethansulfonat-Gruppe, in Gegenwart von Palladium mit der Verbindung der Formel (III): CH₂=CH-R (III),
in der R die Gruppe 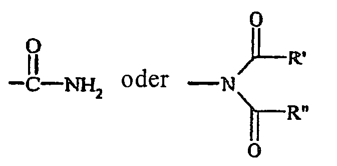 bedeutet, worin R' und R", die identisch oder verschieden sind, jeweils eine geradkettige oder verzweigte (C₁-C₆)-Alkylgruppe bedeuten oder R' und R" gemeinsam eine (C₂-C₃)-Alkylenkette bilden, wobei der in dieser Weise gebildete Ring an einen Phenylrest kondensiert sein kann,
kondensiert zur Bildung der Verbindung der Formel (IV): 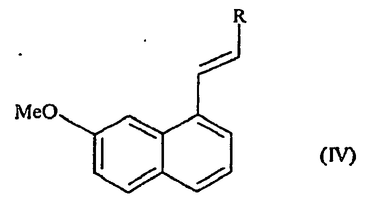 in der R die oben angegebenen Bedeutungen besitzt,
welche man einer katalytischen Hydrierung unterwirft zur Bildung der Verbindung der
Formel (V): 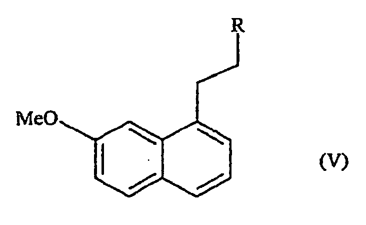 in der R die oben angegebenen Bedeutungen besitzt,
welche man einer basischen oder sauren Hydrolyse unterzieht oder einem binären Reduktionsmittel/Säure-System unterwirft zur Bildung der Verbindung der Formel (VI) oder seinem Hydrochlorid: 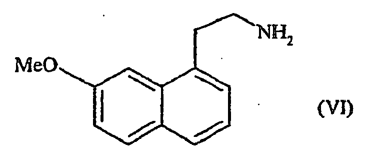 welche nacheinander der Einwirkung von Natriumacetat und dann Essigsäureanhydrid unterworfen wird zur Bildung der Verbindung der Formel (I), welche man in Form eines Feststoffs isoliert.

2. Verfahren zur Synthese der Verbindung der Formel (I) nach Anspruch 1, **dadurch gekennzeichnet, dass** die Verbindung der Formel (III) *N*-Vinylphthalimid ist.

3. Verfahren zur Synthese der Verbindung der Formel (I) nach Anspruch 1, **dadurch gekennzeichnet, dass** die Verbindung der Formel (III) Acrylamid ist.

4. Verfahren zur Synthese der Verbindung der Formel (I) nach Anspruch 1, **dadurch gekennzeichnet, dass** die Kondensation der Verbindung der Formel (III) zur Bildung der Verbindung der Formel (IV) mit Palladium-tetrakistriphenylphosphin durchgeführt wird.

5. Verbindung der Formel (IV) nach Anspruch 1, nützlich als Zwischenprodukt für die Synthese von Agomelatin.

6. Verwendung der Verbindung der Formel (IV) nach Anspruch 5 bei der Synthese von Agomelatin.

7. Verwendung der Verbindung der Formel (II) nach Anspruch 1 bei der Synthese von Agomelatin.

8. Verwendung der Verbindung der Formel (V) nach Anspruch 1 bei der Synthese von Agomelatin, mit der Maßgabe, dass diese Verwendung nicht 2-[2-(7-Methoxy-1-naphthyl)-ethyl]-1*H*-isoindol-1,3(2*H*)-dion betrifft.

9. Verfahren zur Synthese von Agomelatin nach Anspruch 1 ausgehend von der Verbindung der Formel (IV), **dadurch gekennzeichnet, dass** man die Verbindung der Formel (IV) nach dem Syntheseverfahren nach einem der Ansprüche 1 bis 4 herstellt.

10. Verfahren zur Synthese von Agomelatin nach Anspruch 1 ausgehend von der Verbindung der Formel (V), **dadurch gekennzeichnet, dass** man die Verbindung der Formel (V) nach dem Syntheseverfahren nach einem der Ansprüche 1 bis 4 herstellt.

11. Verfahren zur Synthese von Agomelatin nach Anspruch 1 ausgehend von der Verbindung der Formel (VI), **dadurch gekennzeichnet, dass** man die Verbindung der Formel (VI) nach dem Syntheseverfahren nach einem der Ansprüche 1 bis 4 herstellt.
